# FASCICULE DE BREVET EUROPEEN

(11) **EP 0 507 692 B1**
(45) Date de publication et mention de la délivrance du brevet: **04.03.1998**
(21) Numéro de dépôt: 92400933.5
(22) Date de dépôt: 03.04.1992
(51) Int. Cl.: C07D 251/50, C07D 251/52, C07D 251/68, C07D 251/54, C07D 251/70, A61K 7/42

(54) **Dérivés s-triaziniques portant des substituants benzylidène camphre, leur procédé de préparation, compositions cosmétiques filtrantes les contenant et leur utilisation pour protéger la peau et les cheveux du rayonnement ultraviolet**
S-Triazinederivate mit Benzylidencampfer als Substituent, Verfahren zu ihrer Herstellung, diese enthaltende kosmetische Zubereitungen mit filtrierender Wirkung und ihre Verwendung als Schutzmittel für Haut und Haar
S-Triazine derivatives substituted with benzylenecampher, process for their preparation, filtering cosmetic compositions containing them and their use as UV protectors for skin and hair

(30) Priorité: 04.04.1991 FR 9104123
(43) Date de publication de la demande: 07.10.1992
(73) Titulaire: L'OREAL, 75008 Paris (FR)
(72) Inventeur: Richard, Hervé, F-93240 Villepinte (FR); Leduc, Madeleine, F-75011 Paris (FR); Junino, Alex, F-92180 Livry-Gargan (FR)
(74) Mandataire: Casalonga, Axel

(56) Documents cités:
- DE-B- 1 205 970
- GB-A- 2 185 019
- US-A- 4 061 730
- SEIFEN,OLE,FETTE,WACHSE, vol. 115, no. 18, 21 Novembre 1980, AUGSBURG,DE pages 661 - 662; K.SPERLING: 'UV-Filter für Haut-und Produktschutz in kosmetischen Formulierungen'
- RIECHSTOFFE-AROMEN-KOSMETICA.R.A.K, vol. 32, no. 6, Juin 1982, ST.PETER-ORDING, DEUTSCHLAND; I.BECK ET AL.: 'Untersuchungen über das photochemische Verhalten von Sonnenschutzmitteln:Benzylidenkamfer und Derivate'

## Description

La présente invention concerne de nouveaux dérivés s-triaziniques portant des substituants benzylidène camphre, leur procédé de préparation et leur utilisation en tant que filtres solaires, notanment dans le domaine cosmétique.

On sait que les radiations lumineuses de longueurs d'onde comprises entre 280 nm et 400 nm permettent le brunissenment de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage; ce rayonnement UV-B doit donc être filtré.

On sait également que les rayons UV-A de longueurs d'onde comprises entre 320 et 400 nm, provoquant le brunissement de la peau, sont susceptibles d'induire une altération de celle-ci notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques.

Il est donc intéressant de disposer de composés susceptibles d'absorber aussi bien les rayons UV-A que les rayons UV-B nocifs pour la peau.

Il est également souhaitable d'assurer aux cheveux une bonne protection contre la dégradation photochimique afin d'éviter en particulier un changement de nuance ou une décoloration.

On sait par ailleurs que les constituants entrant dans les préparations cosmétiques ne possèdent pas toujours une stabilité suffisante à la lumière et se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement urilisés en cosmétique et, en particulier, dans les huiles et graisses.

On connaît, par le brevet US 4 617 390, des dérivés de s-triazine portant des substituants p-aminobenzoates, qui n'absorbent que le rayonnement UV-B et dont la solubilité dans les corps gras est limitée; il est donc nécessaire, si l'on souhaite absorber l'ensemble du rayonnement UV, d'associer à ces dérivés de s-triazine greffés par des restes p-aminobenzoates, des filtres UV-A.

Par ailleurs, le p-aminobenzylidène camphre, qui est un composé connu, peut être utilisé comme filtre UV-A, mais ce composé est très faiblement soluble dans les corps gras utilisés habituellement en cosmétique.

La demanderesse a découvert que des dérivés s-triaziniques greffés par des restes p-aminobenzylidène camphre, de manière surprenante, absorbaient le rayonnement UV-A ou l'ensemble du rayonnement UV (UV-B et UV-A) et possédaient en même temps une bonne solubilité dans les corps gras. Ces dérivés s-triaziniques possèdent également un coefficient d'absorption molaire très élevé, ce qui permet de les utiliser à faible concentration, par exemple dans les compositions cosmétiques de protection de la peau ou anti-solaires.

De plus, ces nouveaux dérivés s-triaziniques, associés à des filtres UV-A du type dibenzoylméthane, notamment le 4-tert.-butyl-4′-méthoxy-dibenzoylméthane (Parsol 1789), permettent de photostabiliser ces filtres UV-A.

Outre leurs propriétés filtrantes et leur caractère liposoluble, ces nouveaux dérivés s-triaziniques présentent une bonne stabilité chimique et photochimique et ont l'avantage de n'être ni toxiques, ni irritants et d'avoir une parfaite innocuité vis-à-vis de la peau.

La présente invention a donc pour objet les nouveaux composés répondant à la formule suivante (I) : 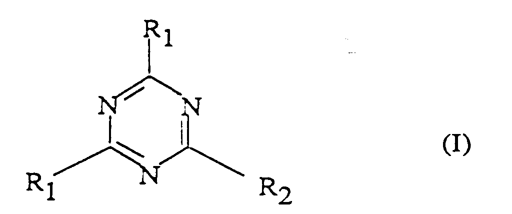 dans laquelle R₁ répond à la formule : 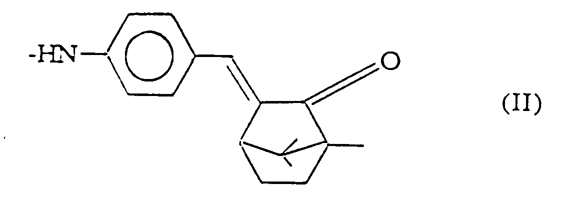 et R₂ est un groupement alcoxy ou mono- ou di-alkylamino linéaire ou ramifié contenant de 8 à 20 atomes de carbone ou répond à la formule (III) : 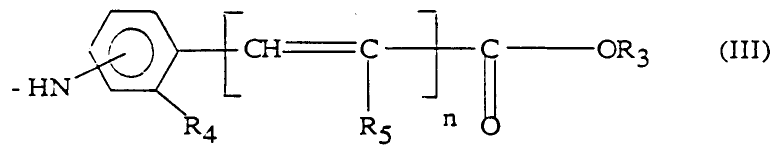 dans laquelle n est égal à 0 ou 1;
- lorsque n est égal à 0, R₃ est un radical alkyle linéaire ou ramifié contenant de 8 à 20 atomes de carbone; R₄ représente un atome d'hydrogène, le reste amino se trouvant en position 4 par rapport au groupement carboxyle, ou R₄ représente un radical hydroxyle ou un radical alcoxy en C₁-C₆, le reste amino se trouvant en position 4 ou 5 par rapport au groupement carboxyle;
- lorsque n est égal à 1, R₃ est un radical alkyle linéaire ou ramifié contenant de 4 à 20 atomes de carbone; R₄ représente un atome d'hydrogène, le reste amino se trouvant en position 4 par rapport au groupement insaturé, R₅ est un atome d'hydrogène ou un groupement COOR₃ dans lequel R₃ a la signification indiquée ci-dessus pour n = 1, sous réserve que lorsque R₅ est un atome d'hydrogène, R₃ doit comporter de 8 à 20 atomes de carbone.

Parmi les radicaux alkyle linéaires ou ramifiés, on peut citer par exemple : n-butyle, isobutyle, tert.-butyle, n-amyle, isoamyle, néopentyle, n-hexyle, n-heptyle, n-octyle, 2-éthylhexyle, n-dodécyle, n-octadécyle.

Parmi les radicaux alcoxy linéaires ou ramifiés, on peut citer par exemple: méthoxy, éthoxy, n-propoxy, isopropoxy, n-butoxy, isobutoxy, tert.-butoxy, n-amyloxy, isoanmyloxy, néopentyloxy, n-hexyloxy, n-heptyloxy, n-octyloxy, 2-éthylhexyloxy, n-dodécyloxy, n-octadécyloxy.

Les composés (I) absorbent le rayonnement UV-A lorsque R₂ est un groupement alcoxy ou mono- ou di-alkylanino ou répond à la fommule (III) dans laquelle n est égal à 1, ou n est égal à 0 et R₄ représente un radical hydroxyle, le reste amino se trouvant en position 5 par rapport au groupement carboxyle, ou absorbent le rayonnement UV large bande (UV-A et UV-B) dans les autres cas.

Ces nouveaux composés s-triaziniques peuvent être utilisés comme filtres solaires pour la peau humaine et les cheveux ainsi que comme agents protecteurs de la lumière dans l'industrie des plastiques.

La présente invention a également pour objet le procédé de préparation des composés de formule (I).

Les composés de formule (I) peuvent être obtenus selon le schéma réactionnel ci-dessous : 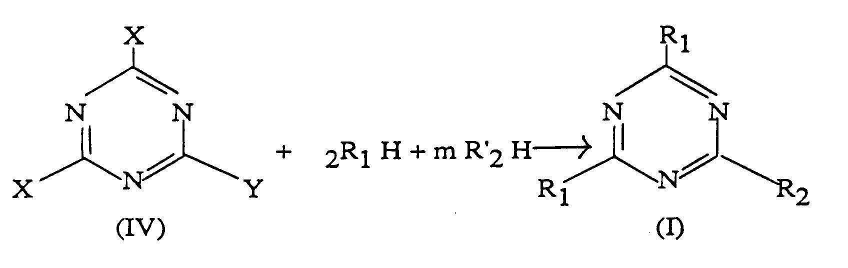 où R₁ répond à la formule (II) indiquée ci-dessus, R′₂ répond à la formule (III) et R₂ a la signification indiquée ci-dessus; X, représente un halogène, en particulier le chlore ou le brome, Y représente X, un groupement alcoxy ou mono- ou di-alkylamino linéaire ou ramifié contenant de 8 à 20 atomes de carbone; m est un nombre entier égal à 1 si Y est égal à X, la réaction s'effectuant en 2 étapes; si Y est différent de X, m est égal à 0.

Les composés de formule générale (IV) dans lesquels Y est différent de X, peuvent être obtenus selon le Schéma réactionnel ci-dessous, qui est décrit dans les publications: J.T. THURSTON et al, J. Am. Chem. Soc., 73, 2981 (1951) et J.R. DUDLEY et al., J. Am. Chem. Soc. 73, 2986 (1951) : 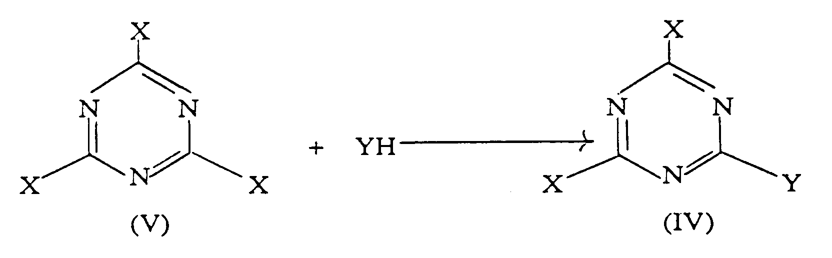 X et Y ayant les mêmes significarions que ci-dessus.

Les réactions peuvent être effectuées éventuellement en présence d'un solvant (par exemple : toluène, xylène ou acétone/eau) à une température entre 0 et 250°C, plus particulièrement entre 0 et 150°C.

Les composés R′₂H où R′₂ a la formule (III) peuvent être préparés selon des méthodes connues décrites dans les brevets FR-2 151 503, FR-A 2 385 685, GB 1 064 116.

Le composé R₁H où R₁ a la formule (II) peut être préparé comme indiqué dans HALLER, BOUDIN, Annales de Chimie, 9e série, tome XVII (1922).

Parmi les composés de formule (I) ci-dessus, on peut citer plus particulièrement les composés suivants:
1) . 2,4-bis(4′-aminobenzylidène camphre)-6-(2-éthylhexylamino)-s-triazine
2) . 2,4-bis(4′-aminobenzylidène camphre)-6-(4′-amino benzalmalonate de diisobutyle)-s-triazine
3) . 2,4-bis(4′-aminobenzylidène camphre)-6-(5′-amino salicylate de 2-éthylhexyle)-s-triazine..

De par leur caractère liposoluble, les composés de formule (I) ci-dessus se répartissent uniformément dans les supports cosmétiques classiques contenant au moins une phase grasse ou un solvant organique cosmétiquement acceptable et peuvent être appliqués sur la peau ou les cheveux pour constituer un film protecteur efficace.

La présente invention a donc aussi pour objet une composition cosmétique comprenant, dans un support cosmétiquement acceptable contenant au mois une phase grasse ou un solvant organique, une quantité efficace d'au moins un composé de formule (I) ci-dessus.

La composition cosmétique de l'invention peut être utilisée comme composition protectrice de l'épiderme humain ou des cheveux ou comme composition anti-solaire.

La présente invention a également pour objet un procédé de protection de la peau et des cheveux naturels ou sensibilisés vis-à-vis du rayonnement solaire, consistant à appliquer sur la peau ou les cheveux une quantité efficace d'une composition cosmétique contenant au moins un composé de formule (I).

On entend par "cheveux sensibilisés" des cheveux ayant subi un traitement de permanente, de coloration ou de décoloration.

L'invention a également pour objet une composition cosmétique colorée ou non colorée, stabilisée à la lumière, comprenant une quantité efficace d'au moins un composé de formule (I) ci-dessus.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotions huileuses ou oléoalcooliques, d'émulsions telles qu'une crème ou un lait, de dispersions vésiculaires de lipides amphiphiles ioniques ou non ioniques, de gels oléoalcooliques ou alcooliques, de bâtonnets solides ou être conditionnée en aérosol. Les crèmes constituent ce que l'on appelle généralement les "crèmes de jour protectrices" d'usage quotidien.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique. Elle peut contenir en outre d'autres filtres UV-A et/ou UV-B.

Le composé de formule (I) est présent dans des proportions en poids comprises entre 0,1 et 2% par rapport au poids total de la composition cosmétique protectrice de l'épiderme humain.

Comme solvant de solubilisation, on peut utiliser une huile, une cire et de façon générale tout corps gras, un monoalcool ou un polyol inférieur ou leurs mélanges. Les monoalcools ou polyols plus particulièrement préférés sont l'éthanol, l'isopropanol, le propylèneglycol, l'hexylèneglycol, la glycérine et le sorbitol.

Une forme de réalisation de l'invention est une émulsion sous forme de crème ou de lait protecteurs comprenant en plus du composé de fommule (I), des alcools gras, des esters d'acides gras et notamment des triglycérides d'acides gras, des acides gras, de la lanoline, des huiles ou cires naturelles ou synthétiques et des émulsionnants, en présence d'eau.

Les dispersions vésiculaires de lipides amphiphiles ioniques ou non-ioniques peuvent être préparées selon des procédés connus.

On peut, par exemple, faire gonfler les lipides dans une solution aqueuse pour former des sphérules dispersées dans le milieu aqueux comme décrit dans l'article BANGHAN, STANDISH et WATKINS, J. Mol. Biol, 13,238 (1965) ou dans les brevets FR-2 315 991 et 2 416 008 de la demanderesse.

Une autre forme de réalisation est constituée par des lotions huileuses à base d'huiles et cires naturelles ou synthétiques, de lanoline, et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylèneglycol ou la glycérine et un épaississant tel que la silice. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

Dans le cas d'une composition conditionnée en aérosol, on utilise les propulseurs classiques.

La présente invention vise également les compositions cosmétiques anti-solaires contenant au moins un composé de formule (I) et pouvant contenir d'autres filtres UV-B et/ou UV-A.

Dans ce cas, la quantité totale de filtres présents dans la composition anti-solaire, c'est-à-dire le composé de formule (I) et éventuellement les autres filtres, est comprise entre 0,3 et 15% en poids par rapport au poids total de la composition anti-solaire.

Ces compositions anti-solaires se présentent sous les formes indiquées ci-dessus pour les compositions protectrices de l'épiderme humain.

A titre de filtres solaires filtrant les rayons UV-B, on peut citer les filtres hydrosolubles tels que les dérivés du benzylidène camphre décrits dans les brevets français 2 199 971, 2 236 515, 2 282 426 et 2 383 904 de la demanderesse et plus particulièrement le méthylsulfate de 4-(2-oxo-3-bornylidène-méthyl)phényltriméthyl ammonium, les sels de l'acide 4-(2-oxo-3-bornylidène méthyl)benzène sulfonique, de l'acide 2-méthyl-5-(2-oxo-3-bornylidèneméthyl)benzène sulfonique, de l'acide 3-benzylidène-2-oxo- 10-bornanesulfonique et de l'acide 2-phénylbenzimidazole-5-sulfonique.

Les composés selon l'invention peuvent également être associés à des filtres UV-B constitués par des composés liposolubles ou par des huiles ayant des propriétés filtrantes telles qu'en particulier l'huile de café. A titre de filtres solaires UV-B lipophiles, on peut citer les dérivés de l'acide salicylique tels que le salicylate de 2-éthylhexyle, le salicylate d'homomenthyle, les dérivés de l'acide cinnamique tels que le p-méthoxycinnamate de 2-éthylhexyle, le p-méthoxycinnamate de 2-éthoxyéthyle, les dérivés de l'acide p-aminobenzoïque tels que le p-aminobenzoate d'amyle, le p-diméthylaminobenzoate de 2-éthyl hexyle, les dérivés de benzophénone tels que la 2-hydroxy 4-méthoxybenzophénone, la 2,2′-dihydroxy 4-méthoxybenzophénone, les dérivés du camphre tels que le 3-(4′-méthylbenzylidène)camphre ou le 3-benzylidènecamphre.

Les composés selon l'invention peuvent aussi être associés à des filtres UV-A parmi lesquels on peut citer les dérivés du dibenzoylméthane, par exemple le 2-méthyl dibenzoylméthane, le 4-méthyldibenzoylméthane, le 4-isopropyldibenzoylméthane, le 4-tert.-butyl-dibenzoylméthane, le 2,4-diméthyldibenzoylméthane, le 2,5-diméthyldibenzoylméthane, le 4,4′-diisopropyldibenzoylméthane, le 4-tert.-butyl-4′-méthoxydibenzoylméthane, le 2-méthyl-5-isopropyl-4′-méthoxydibenzoylméthane, le 2-méthyl-5-tert.-butyl-4′-méthoxydibenzoylméthane, le 2,4-diméthyl-4′-méthoxydibenzoylméthane et le 2,6-diméthyl-4-tert.-butyl-4′-méthoxydibenzoylméthane, ainsi que les dérivés du benzène 1,4-[di(3-méthylidène camphre)] sulfonés sur le radical méthyle en position 10 du camphre tels que décrits dans les brevets français 2 528 420 et 2 639 347.

Il est entendu que la liste de filtres solaires utilisés en association avec les composés (I) selon l'invention qui est indiquée ci-dessus n'est pas limitative.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de laque pour cheveux, de composition de permanente, de coloration ou décoloration des cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousse, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,1 à 2% en poids de composé de formule (I).

La présente invention vise également les compositions cosmétiques contenant au moins un composé de formule (I) à titre d'agent de protection contre les rayons ultraviolets constituées par des compositions capillaires telles que les laques pour cheveux, les lotions de mise en plis éventuellement traitantes ou démêlantes, les shampooings colorants, les compositions tinctoriales pour cheveux; par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière au cours du stockage.

De telles compositions contiennent 0,1 à 2% en poids de composé de formule (I).

Les composés (I) selon l'invention peuvent également être incorporés dans différentes matières organiques, et notamment les matières plastiques, en vue de les protéger contre le rayonnement ultraviolet.

L'invention sera mieux illustrée, sans toutefois être limitée, par les exemples de réalisation suivants.

### EXEMPLE 1

### 2,4-bis(4'-amino benzylidène camphre)-6-(2-éthylhexylamino)-s-triazine

### Préparation de la 2-(2-éthylhexylamino)-4,6-dichloro-s-triazine de départ (IV)

Dans un ballon, on introduit goutte à goutte une solution de chlorure de cyanuryle (0,2 mole, 36,8 g) dans l'acétone (300 ml) à de la glace pilée (100 g) sous vive agitation, puis une solution de 2-éthylhexylamine dans l'acétone (100 ml) en maintenant la température entre 0 et 5°C, et enfin une solution de carbonate de sodium (11,2 g, 0,1 mole) dans 300 ml d'eau. On laisse une heure sous agitation à 0-5°C. On extrait l'huile au dichlorométhane, on lave la phase organique à l'eau et on sèche sur sulfate de sodium. Après évaporation du solvant, on obtient la 2-(2-éthylhexylamino)-4,6-dichloro-s-triazine (52 g, rendement = 95%) ayant pour caractéristiques :
- poudre blanche
- Pf = 37°C

| - Analyse : C₁₁H₁₈Cl₂N₄ | | | | |
|---|---|---|---|---|
| | C% | H% | Cl% | N% |
| Calculé : | 47,66 | 6,55 | 25,58 | 20,21 |
| Trouvé : | 47,89 | 6,60 | 25,79 | 20,16 |

### Préparation du composé (I)

Le dérivé précédent (16,6 g, 0,06 mole) est porté au reflux dans le xylène (300 ml) sous azote pendant 6 heures avec du 4-amino benzylidène camphre (33,7 g, 0,132 mode). Après refroidissement, on filtre le solide et on le lave au xylène et à l'éther diisopropylique. Après deux recristallisations dans l'éthanol absolu, on obtient le composé(20,5 g) ayant pour caractéristiques :
- poudre jaune pâle
- UV : (éthanol à 95°) λₘₐₓ = 354 nm (εₘₐₓ = 67100)

| - Analyse : C₄₅H₅₈N₆O₂ | | | | |
|---|---|---|---|---|
| | C% | H% | N% | O% |
| Calculé : | 75,59 | 8,18 | 11,75 | 4,48 |
| Trouvé : | 75,68 | 8,23 | 11,88 | 4,53 |

### EXEMPLE 2

### 2,4-bis(4'-amino benzylidène camphre)-6-(4'-amino benzalmalonate de diisobutyle)-s-triazine

### 1ère étape

### Préparation de la 2,4-bis(4'-amino benzylidène camphre)-6-chloro-s triazine

A une solution de chlorure de cyanuryle (4,61 g, 0025 mole) dans 50 ml d'acétone on ajoute à 35-40°C, goutte à goutte, le 4-amino benzylidène camphre (12,76 g, 0,05 mode) en solution dans 75 ml d'acétone, puis 50 ml de soude N en 2 heures. L'agitation est maintenue 1,5 heure. Après essorage du précipité, lavage à l'eau et séchage, on obtient la 2,4-bis(4'-amino benzylidène camphre)-6-chloro-s-triazine (12,6 g, rendement = 81%) ayant les caractéristiques suivantes :
- poudre jaune pâle
- Pf = 255°C
- UV = (éthanol à 95°) λₘₐₓ = 346 nm (εₘₐₓ = 61900)

| - Analyse : C₃₇H₄₀ClN₅O₂ | | | | | |
|---|---|---|---|---|---|
| | C% | H% | Cl% | N% | O% |
| Calculé : | 71,42 | 6,48 | 5,70 | 11,26 | 5,14 |
| Trouvé : | 71,43 | 6,51 | 5,66 | 11,15 | 5,35 |

### 2ème étape

### Préparation du composé (I)

Le dérivé précédent (3,11 g, 0,005 mode) et le 4-amino benzalmadonate de diisobutyle (1,6 g, 0,005 mole) sont chauffés au reflux pendant 10 heures sous azote dans 24 ml de xylène. On refroidit, et on neutralise avec une solution saturée de bicarbonate de sodium. La phase organique est lavée à l'eau, séchée sur sulfate de sodium et concentrée. L'huile obtenue est chromatographiée sur colonne de silice (éluant CH₂Cl₂). On obtient le composé (3,5 g, rendement = 78%) ayant les caractéristiques suivantes :
- poudre jaune pâle
- Pf = 186°C
- UV : (éthanol à 95°) = λₘₐₓ = 358nm (εₘₐₓ = 106350)

| - Analyse : C₅₅H₆₄N₆O₆ | | | | |
|---|---|---|---|---|
| | C% | H% | N% | O% |
| Calculé : | 72,98 | 7,13 | 9,28 | 10,61 |
| Trouvé : | 72,68 | 7,18 | 9,11 | 11,01 |

### EXEMPLE 3

### 2,4-bis(4'-amino benzylidène camphre)-6-(5'-amino-salicylate de 2-éthylhexyle)-s-triazine

On porte au reflux pendant 6 heures sous azote la 2,4-bis(4'-amino benzylidène camphre)-6-chloro-s-triazine préparé à la 1ère étape de l'exemple 2 (3,11 g, 0,005 mode) et le 5-amino salicylate de 2-éthylhexyle (1,32 g, 0,005 mode) dans 24 ml de xylène. Après refroidissement, on neutralise avec une solution saturée de bicarbonate de sodium. La phase organique est lavée deux fois à l'eau, séchée sur sulfate de sodium et concentrée. L'huile obtenue est purifiée sur colonne de silice (éluant : CH₂Cl₂). On obtient le produit (3,8 g, rendement = 89%) ayant les caractéristiques suivantes :
- Pf = ramollissement vers 150°C
- UV : (éthanol à 95°) λₘₐₓ = 355 nm (εₘₐₓ = 76340)

| - Analyse : C₅₂H₆₂N₆O₅ | | | | |
|---|---|---|---|---|
| | C% | H% | N% | O% |
| Calculé : | 73,38 | 7,34 | 9,87 | 9,40 |
| Trouvé : | 73,34 | 7,32 | 9,76 | 9,43 |

### EXEMPLE A

### HUILE ANTISOLAIRE

On mélange les ingrédients suivants en chauffant éventuellement à 40-45°C pour homogénéiser :

| | |
|---|---|
| - Huile de vaseline | 2,5 g |
| - Composé de l'exemple 2 | 2,0 g |
| - p-diméthylamino benzoate de 2-éthylhexyle | 4,0 g |
| - Ditert. butyl hydroxy toluène | 0,05 g |
| - Parfum qs | |
| - Triglycérides d'acides gras en C₈-C₁₂ "Miglyol 812" (Société DYNAMIT NOBEL) | 40,0 g |
| - Alcool à 96° qsp | 100,0 g |

### EXEMPLE B

### EMULSION HUILE-DANS-EAU ANTISOLAIRE

| | |
|---|---|
| - Composé de l'exemple 2 | 1,5 g |
| - Paraméthoxycinnamate de 2-éthylhexyle | 2,0 g |
| - Mélange d'alcool cétylstéarylique et cétylstéarylique oxyéthyléné à 33 moles d'oxyde d'éthylène ("SINNOWAX AO") vendu par la Société HENKEL | 7,0 g |
| - Monostéarate de glycérol | 2,0 g |
| - Propylène glycol | 10,0 g |
| - Alcool cétylique | 1,3 g |
| - Benzoate d'alcools gras C₁₂/C₁₅("FINSOLV TN" vendu par la Société WITCO) | 15,0 g |
| - Conservateur | 0,2 g |
| - Parfum qs | |
| - Eau déminéralisée qsp | 100,0 g |

L'émulsion est préparée de la façon suivante :

On dissout le p-méthoxycinnamate de 2-éthylhexyle et le dérivé de l'exemple 2 dans la phase grasse qui est chauffée vers 70-75°C. Par ailleurs, on chauffe à 70-75°C l'eau contenant les composés hydrosolubles et on ajoute la phase grasse à la phase aqueuse. Après 10 minutes d'agitation vive, on laisse refroidir sous agitation modérée et vers 40°C, on ajoute le conservateur et le parfum.

### EXEMPLE C

### HUILE PROTECTRICE DES CHEVEUX

| | |
|---|---|
| - Composé de l'exemple 3 | 1,0 g |
| - Alcool oléique | 19,5 g |
| - Hexylène glycol | 0,5 g |
| - Huile de colza qsp | 100,0 g |

Cette huile, d'aspect opalescent est appliquée sur cheveux séchés auxquels elle apporte de la brillance et de la douceur tout en préservant leur couleur lorsqu'ils sont exposés à la lumière naturelle.

### EXEMPLE D

### CREME CAPILLAIRE PROTECTRICE

| | |
|---|---|
| - Composé de l'exemple 1 | 0,5 g |
| - Alcool cétylstéarylique oxyéthyléné à 33 moles d'oxyde d'éthylène | 4,0 g |
| - Alcool cétylique | 2,0 g |
| - Alcool stéarylique | 2,0 g |
| - Ether de cellulose cationique vendu sous la dénomination "JR 400" par la société UNION CARBIDE | 0,5 g |
| - Eau qsp | 100,0 g |

Cette émulsion est préparée comme dans l'exemple B.

Appliquée sur cheveux mouillés, elle apporte une facitité de démêlage. Les cheveux séchés sont doux et sont protégés du soleil.

### EXEMPLE E

### CREME DE JOUR PROTECTRICE

| | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 7,0 g |
| - Mélange de mono- et distéarate de glycérol non autoémulsionnable | 2,0 g |
| - Alcool cétylique | 1,5 g |
| - Huile de silicone | 1,5 g |
| - Alcool myristique oxypropyléné à 3 moles d'oxyde de propylène vendu sous le nom de "WITCONOL APM" par la Société WITCO | 20,0 g |
| - 4-tert.-butyl-4'-méthoxy dibenzolyméthane vendu sous le nom de "PARSOL 1789" par la Société GIVAUDAN | 1,0 g |
| - Composé de l'exemple 1 | 1,0 g |
| - Glycérine | 20,0 g |
| - Parfum, conservateurs qs | |
| - Eau qsp | 100,0 g |

L'émulsion est préparée de la même façon que dans l'exemple B.

### EXEMPLE F

### CREME DE JOUR PROTECTRICE

| | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 7,0 g |
| - Mélange de mono- et distéarate de glycérol non autoémulsionnable | 2,0 g |
| - Alcool cétylique | 1,5 g |
| - Huile de silicone | 1,5 g |
| - Alcool myristique oxypropyléné à 3 moles d'oxyde de propylène vendu sous le nom de "WITCONOL APM" par la Société WITCO | 20,0 g |
| - Composé de l'exemple 1 | 2,0 g |
| - Glycérine | 20,0 g |
| - Parfum, conservateurs qs | |
| - Eau qsp | 100,0 g |

Cette émulsion est préparée de la même façon que dans l'exemple B.

### EXEMPLE G

### CREME DE JOUR PROTECTRICE

| | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 7,0 g |
| - Mélange de mono- et distéarate de glycérol non autoémulsionnable | 2,0 g |
| - Alcool cétylique | 1,5 g |
| - Huile de silicone | 1,5 g |
| - Alcool myristique oxypropyléné à 3 moles d'oxyde de propylène vendu sous le nom de "WITCONOL APM" par la Société WITCO | 20,0 g |
| - Glycérine | 20,0 g |
| - Acide benzène 1,4-[di(3-méthylidène-10-camphosulfonique)] | 1,0g MA |
| - Composé de l'exemple 1 | 1,0 g |
| - Triétanolamine | 0,6 g |
| - Parfum, conservateurs qs | |
| - Eau purifiée qsp | 100,0 g |

Cette émulsion huile-dans-eau est préparée de la même façon que dans l'exemple B.

## Revendications

1. Composé de formule : 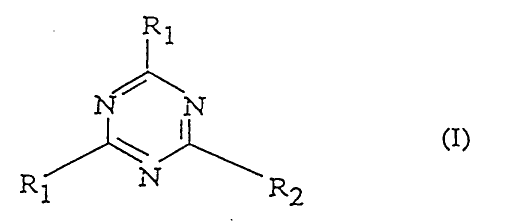 dans laquelle R₁ répond à la formule : 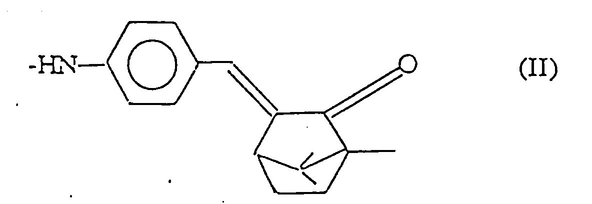 et R₂ est un groupement alcoxy ou mono- ou di-alkylamino linéaire ou ramifié contenant de 8 à 20 atomes de carbone ou répond à la formule (III) : 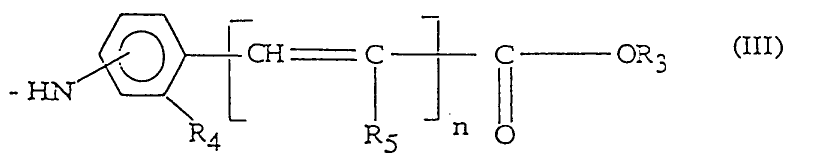 dans laquelle n est égal à 0 ou 1;
- lorsque n est égal à 0, R₃ est un radical alkyle linéaire ou ramifié contenant de 8 à 20 atomes de carbone; R₄ représente un atome dhydrogène, le reste amino se trouvant en position 4 par rapport au groupement carboxyle, ou R₄ représente un radical hydroxyle ou un radical alcoxy en C₁-C₆, le reste amino se trouvant en position 4 ou 5 par rapport au groupement carboxyle;
- lorsque n est égal à 1 R₃ est un radical alkyle linéaire ou ramifié contenant de 4 à 20 atomes de carbone, R₅ est un atome d'hydrogène ou un groupement COOR₃ où R₃ est un radical alkyle linéaire ou ramifié qui contient 4 à 20 atomes de carbone; R₄ représente un atome d'hydrogène, le reste amino se trouvant en position 4 par rapport au groupement insaturé, sous réserve que lorsque R₅ est un atome d'hydrogène, R₃ doit comporter de 8 à 20 atomes de carbone.

2. Composé selon la revendication 1, caractérisé par le fait qu'il est choisi parmi les composés suivants :
1) . 2,4-bis(4'-amino benzylidène camphre)-6-(2-éthylhexylamino)-s-triazine
2) . 2,4-bis(4'-amino benzylidène camphre)-6-(4'-amino benzalmalonate de diisobutyle)-s-triazine
3) . 2,4-bis(4'-amino benzylidène camphre)-6-(5'-amino salicylate de 2-éthylhexyle)-s-triazine.

3. Procédé de préparation du composé de formule (I) selon la revendication 1 ou 2, caractérisé par le fait qu'il est mis en oeuvre selon le schéma réactionnel suivant : 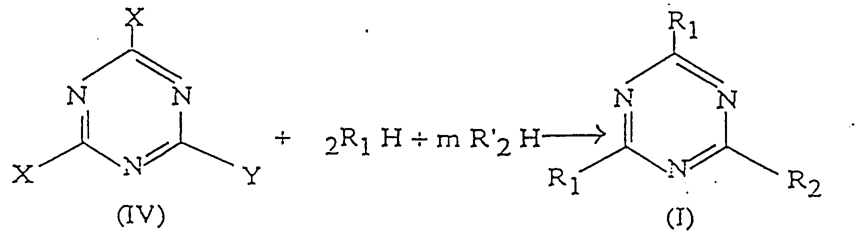 où R₁ répond à la formule (II) indiquée dans la revendication 1 et R'₂ répond à la formule (III) de la revendication 1; X représente un halogène; Y représente X, un groupement alcoxy ou mono- ou dialkylamino linéaire ou ramifié contenant de 8 à 20 atomes de carbone; m est un nombre entier égal à 1 si Y est égal à X et égal à 0 dans le cas contraire,
la réaction étant effectuée éventuellement en présence d'un solvant tel que le toluène, le xylène ou un mélange acétone/eau, à une température comprise entre 0 et 250°C, de préférence entre 0 et 150°C.

4. Composition cosmétique caractérisée par le fait qu'elle comprend une quantité efficace d'au moins un composé de formule (I) selon la revendication 1 ou 2, dans un support cosmétiquement acceptable contenant au moins une phase grasse ou un solvant organique.

5. Composition cosmétique selon la revendication 4, caractérisée par le fait qu'elle comprend au moins un des composés choisis parmi les composés suivants :
1) . 2,4-bis(4'-amino benzylidène camphre)-6-(2-éthylhexylamino)-s-triazine
2) . 2,4-bis(4'-amino benzylidène camphre)-6-(4'-amino benzalmalonate de diisobutyle)-s-triazine
3) . 2,4-bis(4'-amino benzylidène camphre)-6-(5'-amino salicylate de 2-éthylhexyle)-s-triazine.

6. Composition cosmétique selon la revendication 4 ou 5, caractérisée par le fait qu'elle se présente, sous forme de lotion huileuse ou oléoalcoolique, d'émulsion, de gel oléoalcoolique ou alcoolique,de dispersion vésiculaire, de bâtonnet solide ou d'aérosol.

7. Composition cosmétique selon la revendication 6, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, propulseurs, colorants et pigments.

8. Composition cosmétique protectrice de l'épiderme humain selon l'une quelconque des revendications 4 à 7, caractérisée par le fait qu'elle contient 0,1 à 2% en poids de composé de formule (I).

9. Composition cosmétique anti-solaire selon l'une quelconque des revendications 4 à 7, caractérisée par le fait qu'elle contient 0,3 à 15% en poids de composé de formule (I).

10. Composition cosmétique selon la revendication 8 ou 9, caractérisée par le fait qu'elle contient en outre un agent filtrant les rayons UV-B et/ou UV-A différent du composé de formule (I).

11. Composition cosmétique selon la revendication 10, caractérisée par le fait que l'agent filtrant les rayons UV-A et/ou UV-B est un filtre UV-A du type dibenzoylméthane.

12. Composition cosmétique selon la revendication 4 ou 5, destinée à être appliquée sur les cheveux, caractérisée par le fait qu'elle se présente sous forme de shampooing, lotion, gel ou émulsion à rincer, lotion ou gel coiffants ou traitants, lotion ou gel pour le brushing ou la mise en plis, laque pour cheveux, composition de permanente, de décoloration ou coloration et comprend 0,1 à 2% en poids de composé de formule (I).

13. Composition cosmétique selon la revendication 4 ou 5, sous forme d'une composition cosmétique stabilisée à la lumière, caractérisée par le fait qu'elle est constituée par une composition capillaire, un produit de maquillage ou une composition pour les soins ou pour le traitement de la peau, comprenant 0,1 à 2% en poids de composé de formule (I).

14. Utilisation d'un composé de formule (I) selon la revendication 1 ou 2 pour photostabiliser un filtre UV-A de type dibenzoylméthane.

15. Procédé de protection de la peau et des cheveux naturels ou sensibilisés contre le rayonnement ultraviolet, caractérisé par le fait qu'il consiste à appliquer sur la peau ou les cheveux une quantité efficace d'une composition cosmétique contenant au moins un composé de formule (I) selon la revendication 1 ou 2.

16. Composition cosmétique selon la revendication 10, caractérisée par le fait que l'agent filtrant les rayons UV-A est le 4-tert.-butyl-4'-méthoxydibenzoyl-méthane.

17. Utilisation d'un composé de formule (I) selon la revendication 1 ou 2 pour photostabiliser le 4-tert.-butyl-4'-méthoxydibenzoyl-méthane.

## Claims

1. Compound of formula: 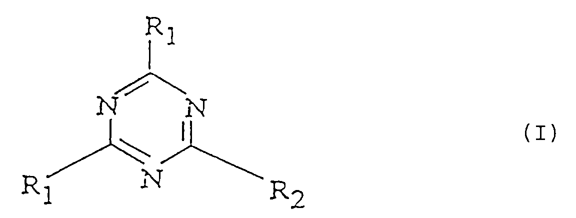 in which R₁ is of the formula: 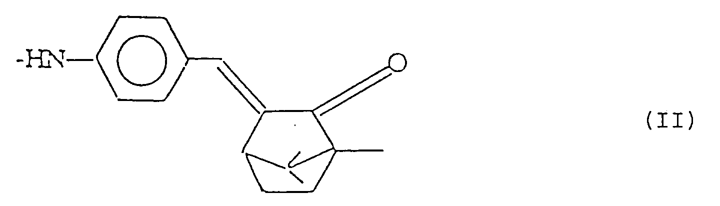 and R₂ is a linear or branched alkoxy or mono- or dialkylamino group containing 8 to 20 carbon atoms or is of the formula (III): 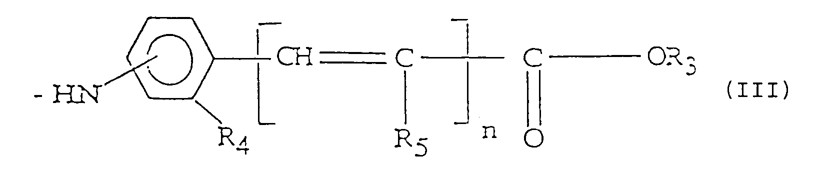 in which n is equal to 0 or 1;
- when n is equal to 0, R₃ is a linear or branched alkyl radical containing 8 to 20 carbon atoms; R₄ represents a hydrogen atom, the amino residue being in position 4 with respect to the carboxyl group, or R₄ represents a hydroxyl radical or a C₁-C₆ alkoxy radical, the amino residue being in position 4 or 5 with respect to the carboxyl group;
- when n is equal to 1, R₃ is a linear or branched alkyl radical containing 4 to 20 carbon atoms, R₅ is a hydrogen atom or a group COOR₃ where R₃ is a linear or branched alkyl radical which contains 4 to 20 carbon atoms; R₄ represents a hydrogen atom, the amino residue being in position 4 with respect to the unsaturated group, provided that when R₅ is a hydrogen atom, R₃ should contain 8 to 20 carbon atoms.

2. Compound according to Claim 1, characterised in that it is chosen from the following compounds:
1) • 2,4-bis(4'-aminobenzylidenecamphor)-6-(2-ethylhexylamino)-s-triazine
2) • 2,4-bis(4'-aminobenzylidenecamphor)-6-(diisobutyl 4'-aminobenzalmalonate)-s-triazine
3) • 2,4-bis(4'-aminobenzylidencamphor)-6-(2-ethylhexyl 5'-aminosalicylate)-s-triazine.

3. Method of preparing the compound of formula (I) according to Claim 1 or 2, characterised in that it is carried out according to the following reaction scheme: 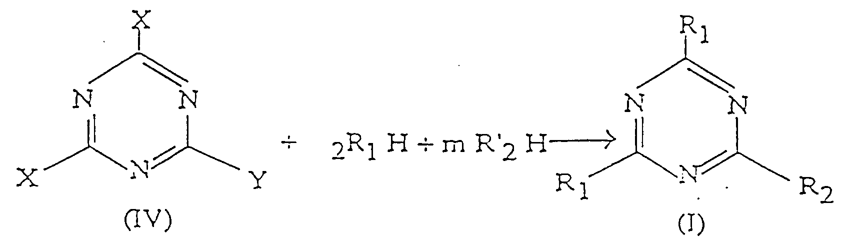 where R₁ is of the formula (II) indicated in Claim 1 and R'₂ is of the formula (III) of Claim 1; X represents a halogen; Y represents X, a linear or branched alkoxy or mono- or dialkylamino group containing 8 to 20 carbon atoms; m is an integer equal to 1 if Y is equal to X and equal to 0 if it is not, the reaction optionally being carried out in the presence of a solvent such as toluene, xylene or an acetone/water mixture, at a temperature between 0 and 250°C, preferably between 0 and 150°C.

4. Cosmetic composition characterised in that it contains an effective amount of at least one compound of formula (I) according to Claim 1 or 2, in a cosmetically acceptable carrier containing at least one fatty phase or one organic solvent.

5. Cosmetic composition according to Claim 4, characterised in that it contains at least one of the compounds chosen from the following compounds:
1) • 2,4-bis (4'-aminobenzylidenecamphor)-6-(2-ethylhexylamino)-s-triazine
2) • 2,4-bis(4'-aminobenzylidenecamphor)-6-(diisobutyl 4'-aminobenzalmalonate)-s-triazine
3) • 2,4-bis(4'-aminobenzylidencamphor)-6-(2-ethylhexyl 5'-aminosalicylate)-s-triazine.

6. Cosmetic composition according to Claim 4 or 5, characterised in that it is provided in the form of an oily or oil-alcohol lotion, emulsion, oil-alcohol or alcoholic gel, vesicular dispersion, solid stick or aerosol.

7. Cosmetic composition according to Claim 6, characterised in that it contains in addition cosmetic adjuvants chosen from thickeners, demulcents, humectants, surface-active agents, preservatives, antifoams, perfumes, oils, waxes, lanolin, lower monoalcohols and polyols, propellants, colorants and pigments.

8. Cosmetic human epidermis-protecting composition according to any one of Claims 4 to 7, characterised in that it contains 0.1 to 2 % by weight of the compound of formula (I).

9. Cosmetic antisun composition according to any one of Claims 4 to 7, characterised in that it contains 0.3 to 15 % by weight of the compound of formula (I).

10. Cosmetic antisun composition according to Claim 9, characterised in that it contains in addition an agent screening out UV-B and/or UV-A rays which is different from the compound of formula (I).

11. Cosmetic composition according to Claim 10, characterised in that the agent screening out UV-A and/or UV-B rays is a UV-A screening agent of the dibenzoylmethane type.

12. Cosmetic composition according to Claim 4 or 5, intended to be applied to hair, characterised in that it is provided in the form of a rinse-off shampoo, lotion, gel or emulsion, hair styling or treating lotion or gel, blow drying or hair setting lotion or gel, hair lacquer, permanent wave, bleaching or dyeing composition, and contains 0.1 to 2 % by weight of the compound of formula (I).

13. Cosmetic composition according to Claim 4 or 5, in the form of a cosmetic composition stabilised with respect to light, characterised in that it consists of a hair composition, a make-up product or a skin care or treatment composition, containing 0.1 to 2 % by weight of the compound of formula (I).

14. Use of a compound of formula (I) according to Claim 1 or 2 to photostabilise a UV-A screening agent of dibenzoylmethane type.

15. Method of protecting the skin and natural or sensitised hair against ultraviolet radiation, characterised in that it consists in applying to the skin or the hair an effective amount of a cosmetic composition containing at least one compound of formula (I) according to Claim 1 or 2.

16. Cosmetic composition according to Claim 10, characterised in that the agent screening out UV-A rays is 4-tert-butyl-4'-methoxydibenzoylmethane.

17. Use of a compound of formula (I) according to Claim 1 or 2 to photostabilise 4-tert-butyl-4'-methoxydibenzoylmethane.

## Patentansprüche

1. Verbindung der Formel: 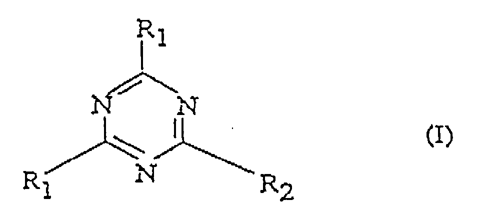 worin R₁' die Formel aufweist: 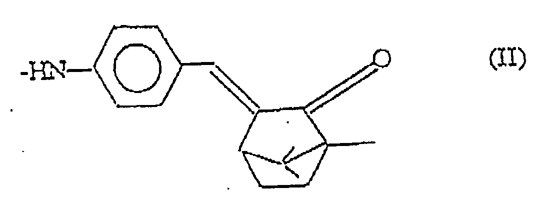 und R₂ eine lineare oder verzweigte Alkoxy- oder Mono- oder Dialkylaminogruppe mit 8 bis 20 Kohlenstoffatomen ist oder die Formel (III) aufweist: 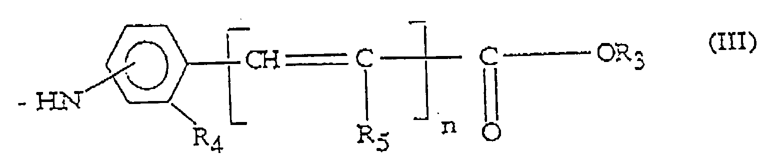 worin n gleich 0 oder 1 ist;
und worin ferner gilt:
- wenn n gleich 0 ist, stellen R₃ einen linearen oder verzweigten Alkylrest mit 8 bis 20 Kohlenstoffatomen, R₄ ein Wasserstoffatom, wobei der Aminorest in Position 4 vorliegt, bezogen auf die Carboxylgruppierung, oder einen Hydroxyl- oder C₁₋₆-Alkoxyrest dar, wobei der Aminorest in Position 4 oder 5 vorliegt, bezogen auf die Carboxylgruppierung;
- wenn n gleich 1 ist, sind R₃ ein linearer oder verzweigter Alkylrest mit 4 bis 20 Kohlenstoffatomen, R₅ ein Wasserstoffatom oder eine COOR₃-Gruppierung, worin R₃ ein linearer oder verzweigter Alkylrest mit 4 bis 20 Kohlenstoffaotmen ist, und R₄ ein Wasserstoffatom, wobei der Aminorest in Position 4 vorliegt, bezogen auf die ungesättigte Gruppierung, mit der Maßgabe, daß, wenn R₅ ein Wasserstoffatom ist, R₃ 8 bis 20 Kohlenstoffatome aufweisen muß.

2. Verbindung gemäß Anspruch 1,
dadurch **gekennzeichnet**, daß
sie aus den folgenden Verbindungen ausgewählt ist:
1) 2,4-Bis(4'-aminobenzylidenkampfer)-6-(2-ethylhexylamino)-s-triazin;
2) 2,4-Bis(4'-aminobenzylidenkampfer)-6-(diisobutyl-4'-aminobenzalmalonat)-s-triazin;
3) 2,4-Bis(4'-aminobenzylidenkampfer)-6-(2-ethylhexyl-5'-aminosalicylat)-s-triazin.

3. Verfahren zur Herstellung der Verbindung der Formel (I) gemäß Anspruch 1 oder 2,
dadurch **gekennzeichnet**, daß
es gemäß dem folgenden Reaktionsschema durchgeführt wird: 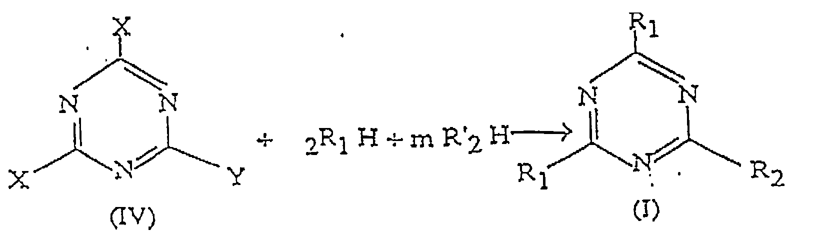 worin R₁ die im Anspruch 1 angegebene Formel (II) und R'₂ die Formel (III) des Anspruchs 1 aufweisen, X ein Halogen und Y X, eine lineare oder verzweigte Alkoxy- oder Mono- oder Dialkylaminogruppierung mit 8 bis 20 Kohlenstoffatomen darstellen und m eine ganze Zahl gleich 1, wenn Y gleich X ist, und gleich 0 in den anderen Fällen ist,
wobei die Reaktion gegebenenfalls in Gegenwart eines Lösungsmittels wie Toluol, Xylol oder einer Mischung aus Aceton/Wasser bei einer Temperatur von 0 bis 250 und vorzugsweise von 0 bis 150°C durchgeführt wird.

4. Kosmetische Zusammensetzung,
dadurch **gekennzeichnet**, daß
sie in einer wirksamen Menge mindestens eine Verbindung der Formel (I) gemäß Anspruch 1 oder 2 in einem kosmetisch geeigneten Träger umfaßt, der mindestens eine Fettphase oder ein organisches Lösungsmittel enthält.

5. Kosmetische Zusammensetzung gemäß Anspruch 4,
dadurch **gekennzeichnet**, daß
sie mindestens eine der Verbindungen enthält, die aus den folgenden Verbindungen ausgewählt sind:
1) 2,4-Bis(4'-aminobenzylidenkampfer)-6-(2-ethylhexylamino)-s-triazin;
2) 2,4-Bis(4'-aminobenzylidenkampfer)-6-(diisobutyl-4'-aminobenzalmalonat)-s-triazin;
3) 2,4-Bis(4'-aminobenzylidenkampfer)-6-(2-ethylhexyl-5'-aminosalicylat)-s-triazin.

6. Kosmetische Zusammensetzung gemäß Anspruch 4 oder 5,
dadurch **gekennzeichnet**, daß
sie in Form einer öligen oder oleoalkoholischen Lotion, einer Emulsion, eines oleoalkoholischen oder alkoholischen Gels, einer bläschenartigen Dispersion, eines Feststoffstäbchens oder eines Aerosol vorliegt.

7. Kosmetische Zusammensetzung gemäß Anspruch 6,
dadurch **gekennzeichnet**, daß
sie ausserdem kosmetische Hilfsstoffe enthält, die aus Verdickungsmitteln, weichmachenden Mitteln, Feuchtigkeitsmitteln, oberflächenaktiven Mitteln, Konservierungsstoffen, Antischaummitteln, Parfüm-Produkten, Ölen, Wachsen, Lanolin, Niedrigmonoalkoholen und -polyolen, Treibmitteln, Farbstoffen und Pigmenten ausgewählt sind.

8. Zum Schutz der menschlichen Epidermis vorgesehene kosmetische Zusammensetzung gemäß jedem der Ansprüche 4 bis 7,
dadurch **gekennzeichnet**, daß
sie 0,1 bis 2 Gew.% Verbindung der Formel (I) enthält.

9. Kosmetische Sonnenschutzmittelzusammensetzung gemäß jedem der Ansprüche 4 bis 7,
dadurch **gekennzeichnet**, daß
sie 0,3 bis 15 Gew.% Verbindung der Formel (I) enthält.

10. Kosmetische Zusammensetzung gemäß Anspruch 8 oder 9,
dadurch **gekennzeichnet**, daß
sie ausserdem ein Filtermittel für die UV-B- und/oder UV-A-Strahlen enthält, welches sich von der Verbindung der Formel (I) unterscheidet.

11. Kosmetische Zusammensetzung gemäß Anspruch 10,
dadurch **gekennzeichnet**, daß
das Mittel, das die UV-A- und/oder UV-B-Strahlen filtert, ein UV-A-Filter vom Dibenzoylmethan-Typ ist.

12. Kosmetische Zusammensetzung gemäß Anspruch 4 oder 5 zur Aufbringung auf die Haare,
dadurch **gekennzeichnet**, daß
sie in Form eines Shampoo, einer Lotion, eines Gel oder einer Emulsion zur Spülung, einer Frisier- oder Behandlungslotion oder eines entsprechenden Gel, einer Lotion oder eines Gel zum Ausbürsten oder zur Wellengebung, eines Lacks für die Haare und einer Zusammensetzung für Dauerwelle, Entfärbung oder Färbung vorliegt und 0,1 bis 2 Gew.% Verbindung der Formel (I) enthält.

13. Kosmetische Zusammensetzung gemäß Anspruch 4 oder 5 in Form einer kosmetischen Zusammensetzung, die gegenüber der Einwirkung von Licht stabilisiert ist,
dadurch **gekennzeichnet**, daß
sie eine Haarmittelzusammensetzung, ein Schminkprodukt oder eine Zusammensetzung für Pflegemaßnahmen oder für die Behandlung der Haut darstellt und 0,1 bis 2 Gew.% Verbindung der Formel (I) enthält.

14. Verwendung einer Verbindung der Formel (I) gemäß Anspruch 1 oder 2 zur Fotostabilisierung eines UV-A-Filters vom Dibenzoylmethan-Typ.

15. Verfahren zum Schutz der Haut oder natürlicher oder sensibilisierter Haare vor der ultravioletten Strahlung,
dadurch **gekennzeichnet**, daß
man auf die Haut oder die Haare eine wirksame Menge einer kosmetischen Zusammensetzung aufbringt, die mindestens eine Verbindung der Formel (I) gemäß Anspruch 1 oder 2 enthält.

16. Kosmetische Zusammenetzung gemäß Anspruch 10,
dadurch **gekennzeichnet**, daß
das Filtermittel für die UV-A-Strahlen 4-t-Butyl-4'-methoxydibenzoylmethan ist.

17. Verwendung einer Verbindung der Formel (I) gemäß Anspruch 1 oder 2 zur Fotostabilisierung von 4-t-Butyl-4'-methoxydibenzoylmethan.
